# EUROPÄISCHE PATENTSCHRIFT

(11) **EP 3 334 344 B1**
(45) Veröffentlichungstag und Bekanntmachung des Hinweises auf die Patenterteilung: **07.07.2021**
(21) Anmeldenummer: 16753853.7
(22) Anmeldetag: 01.08.2016
(51) Int. Cl.: A61B 6/00, A61B 90/90

(54) **RÖNTGENSYSTEM MIT EINEM RÖNTGENSTRAHLER**
X-RAY SYSTEM COMPRISING AN X-RAY SOURCE
SYSTÈME DE RADIOGRAPHIE À RAYONS X

(30) Priorität: 12.08.2015 DE 102015215377
(43) Veröffentlichungstag der Anmeldung: 20.06.2018
(73) Patentinhaber: Siemens Healthcare GmbH, 91052 Erlangen (DE)
(72) Erfinder: FÜRSTENBERG, Dieter, 91052 Erlangen (DE); STEIGER, Klaus, 91080 Uttenreuth (DE)
(86) Internationale Anmeldenummer: PCT/EP2016/068298
(87) Internationale Veröffentlichungsnummer: WO 2017/025369

(56) Entgegenhaltungen:
- DE-A1- 10 057 626
- DE-A1-102006 036 832
- DE-A1-102013 206 146
- DE-T2- 69 915 680
- US-A1- 2011 013 220

## Beschreibung

Die vorliegende Erfindung betrifft ein Röntgensystem mit einem Röntgenstrahler und ein Verfahren zum Betreiben eines Röntgensystems.

Röntgenstrahler für Röntgengeräte werden von unterschiedlichen Herstellern und in unterschiedlichen Fabrikaten angeboten. Allerdings ist die Kompatibilität dieser Röntgenstrahler untereinander nicht immer gegeben. Werden ungeeignete Röntgenstrahler anderer Hersteller in das Röntgengerät eingesetzt, können Fehlfunktionen auftreten oder das Röntgengerät beschädigt werden.

Aus der Druckschrift DE 10057626 A1 ist bekannt, Röntgengeräte vor entweder einer unrichtigen Installation einer Röntgenröhre oder einem Versuch, eine Röhre zu installieren und zu betreiben, die für einen Betrieb an dem fraglichen Abbildungssystem nicht richtig gekennzeichnet wurde, zu schützen. Aus der Druckschrift US 2011/013220 A1 ist weiterhin bekannt, zu überprüfen, ob eine in einem modularen Röntgengerät verwendete Röntgenquelle und ein verwendeter Röntgendetektor kompatibel sind. Aus der Druckschrift DE 10 2006 036832 A1 ist außerdem bekannt, ein Detektormodul aus einer Vielzahl an Detektormodulen mittels einer Signatur zu identifizieren.

Es ist daher die der Erfindung zugrundeliegende Aufgabe, den Einsatz von ungeeigneten Röntgenstrahlern in einem Röntgensystem zu verhindern.

Diese Aufgabe wird durch Gegenstände nach den unabhängigen Ansprüchen gelöst. Vorteilhafte Ausführungsformen sind Gegenstand der abhängigen Ansprüche, der Beschreibung und der Zeichnungen.

Gemäß einem ersten Aspekt wird die Aufgabe durch ein Röntgensystem gelöst, mit einem Röntgenstrahler mit einem elektronisch auslesbaren Datenträger, auf dem ein Identifikationscode gespeichert ist; einer Abfrageeinrichtung zum elektronischen Abfragen des Identifikationscodes aus dem Datenträger des Röntgenstrahlers, wobei der Datenträger eine elektronische Signatur zum Überprüfen der Integrität des Identifikationscodes umfasst; und einer Sperreinrichtung zum Sperren eines Betriebs des Röntgenstrahlers, falls der abgefragte Identifikationscode von einem vorgegebenen Identifikationscode abweicht. Dadurch wird der technische Vorteil erreicht, dass lediglich passende Röntgenstrahler in das Röntgensystem einsetzbar sind, die anhand des Identifikationscodes automatisch identifiziert werden können. Eine Gefährdung des Personals oder eine Beschädigung des Röntgensystems durch einen Einbau unpassender oder ungeeigneter Röntgenstrahler kann ausgeschlossen werden.

In einer vorteilhaften Ausführungsform des Röntgensystems ist der Datenträger in dem Röntgenstrahler integriert. Dadurch wird beispielsweise der technische Vorteil erreicht, dass der Datenträger ein integraler Bestandteil des Röntgenstrahlers ist und nur zusammen mit diesem ausgetauscht werden kann.

In einer weiteren vorteilhaften Ausführungsform des Röntgensystems ist der Datenträger ein Speicherchip. Dadurch wird beispielsweise der technische Vorteil erreicht, dass der Identifikationscode auf einfache Weise gespeichert und ausgelesen werden kann.

In einer weiteren vorteilhaften Ausführungsform des Röntgensystems ist der Identifikationscode in dem elektronisch auslesbaren Datenträger festverdrahtet gespeichert. Dadurch wird beispielsweise der technische Vorteil erreicht, dass der Identifikationscode nachträglich nicht verändert werden kann.

Der Datenträger umfasst eine elektronische Signatur zum Überprüfen der Integrität des Identifikationscode. Dadurch wird beispielsweise der technische Vorteil erreicht, dass der Identifikationscode nicht gefälscht werden kann.

In einer weiteren vorteilhaften Ausführungsform des Röntgensystems umfasst das Röntgensystem eine Empfangseinheit zum Empfangen des vorgegebenen Identifikationscodes von einem Lizenzserver über ein Datennetz. Dadurch wird beispielsweise der technische Vorteil erreicht, dass ein Identifikationscode zum Freischalten des Röntgenstrahlers angefordert werden kann. Dadurch kann gegebenenfalls ein Röntgenstrahler verwendet werden, der ein anderes Fabrikat aufweist.

In einer weiteren vorteilhaften Ausführungsform des Röntgensystems weist der vorgegebene Identifikationscode eine zeitlich begrenzte Gültigkeit auf. Dadurch wird beispielsweise der technische Vorteil erreicht, dass der Röntgenstrahler lediglich in einem bestimmten Zeitraum verwendbar ist.

Gemäß einem zweiten Aspekt könnte die Aufgabe durch einen Röntgenstrahler mit einem elektronisch auslesbaren Datenträger gelöst werden, auf dem ein Identifikationscode für den Röntgenstrahler gespeichert ist. Dadurch werden die gleichen technischen Vorteile wie durch das Röntgensystem nach dem ersten Aspekt erreicht.

In einer vorteilhaften Ausführungsform des Röntgenstrahlers ist der Datenträger in dem Röntgenstrahler integriert. Dadurch wird beispielsweise ebenfalls der technische Vorteil erreicht, dass der Datenträger ein integraler Bestandteil des Röntgenstrahlers ist und nur zusammen mit diesem ausgetauscht werden kann.

Gemäß einem dritten Aspekt wird die Aufgabe durch ein Verfahren zum Betreiben eines Röntgensystem gelöst, mit den Schritten eines elektronischen Abfragens eines Identifikationscodes aus einem Datenträger eines Röntgenstrahlers, wobei der Datenträger eine elektronische Signatur zum Überprüfen der Integrität des Identifikationscodes umfasst; und eines Sperrens eines Betriebs des Röntgenstrahlers, falls der abgefragte Identifikationscode von einem vorgegebenen Identifikationscode abweicht. Dadurch werden die gleichen technischen Vorteile wie durch das Röntgensystem nach dem ersten Aspekt erreicht.

In einer vorteilhaften Ausführungsform des Verfahrens wird die Integrität des abgefragten Identifikationscodes anhand einer elektronischen Signatur überprüft. Dadurch wird beispielsweise ebenfalls der technische Vorteil erreicht, dass der Identifikationscode nicht gefälscht werden kann.

In einer weiteren vorteilhaften Ausführungsform des Verfahrens wird der vorgegebene Identifikationscode von einem Lizenzserver über ein Datennetz empfangen. Dadurch wird beispielsweise ebenfalls der technische Vorteil erreicht, dass ein passender Identifikationscode von dem Lizenzserver angefordert werden kann.

In einer weiteren vorteilhaften Ausführungsform des Verfahrens wird beim Sperren des Betriebes des Röntgenstrahlers eine Nachricht an den Lizenzserver gesendet. Dadurch wird beispielsweise der technische Vorteil erreicht, dass zentral erfasst wird, wenn ein Röntgenstrahler gesperrt wird und ein Service des Röntgensystems durchgeführt werden kann.

In einer weiteren vorteilhaften Ausführungsform des Verfahrens wird das Verfahren vor dem Einstellen der Betriebsparameter des Röntgenstrahlers in dem Röntgensystem durchgeführt. Dadurch wird beispielsweise der technische Vorteil erreicht, dass ein Betrieb des Röntgenstrahlers verhindert wird.

Gemäß einem vierten Aspekt wird die Aufgabe durch ein Computerprogramm mit Programmcode zum Ausführen des Verfahrens nach dem ersten Aspekt gelöst, wenn das Computerprogramm auf einem Computer mit einem Speicher und einem Prozessor, welcher Bestandteil des Röntgensystems ist, ausgeführt wird. Dadurch werden die gleichen technischen Vorteile wie durch das Röntgensystem nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Darstellung eines Röntgensystems; und
- Fig. 2: ein Blockdiagram des Verfahrens zum Betreiben eines Röntgensystems.

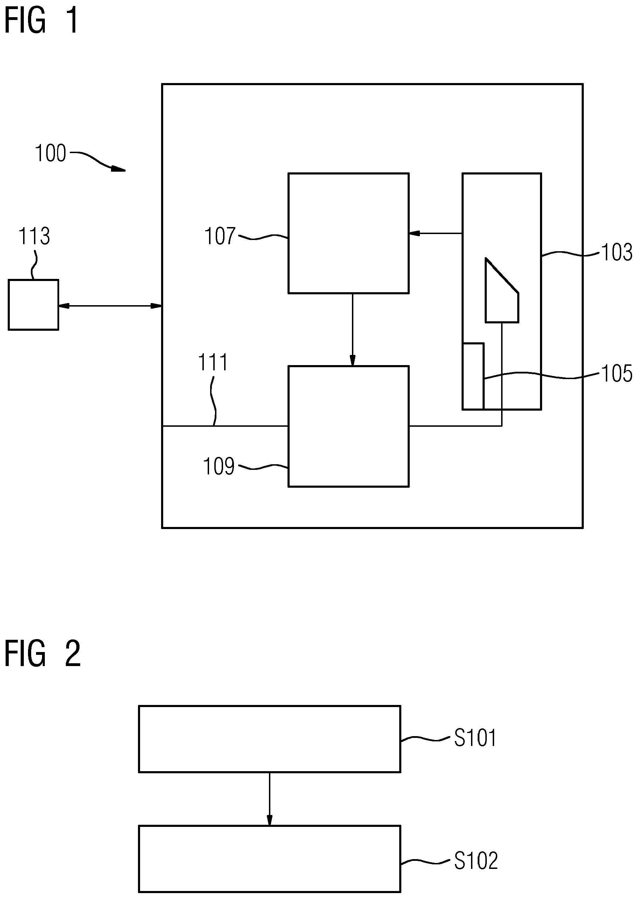

Fig. 1 zeigt eine schematische Darstellung eines Röntgensystems 100. Das Röntgensystem 100 dient zum Untersuchen von Objekten mittels Röntgenstrahlen. Die Röntgenstrahlen werden von einem Röntgenstrahler 103 emittiert, der in das Röntgensystem 100 einsetzbar ist und austauschbar ist. Der Röntgenstrahler 103 umfasst eine Röntgenröhre und ein Schutzgehäuse. Nach dem Durchstrahlen des Objektes treffen die Röntgenstrahlen auf einen Detektor, so dass eine Röntgenaufnahme von dem Objekt erhalten wird.

Zusätzlich umfasst der Röntgenstrahler 103 einen elektronisch auslesbaren Datenträger 105, auf dem ein Identifikationscode zur Erkennung des Röntgenstrahlers 103 gespeichert ist. Daneben kann der Datenträger 105 eine Materialnummer und/oder eine Seriennummer der Röntgenröhre umfassen. Der Datenträger 105 ist beispielsweise ein Speicherchip, auf dem der Identifikationscode digital gespeichert ist. Die Speicherung des Identifikationscodes kann durch eine festverdrahtete Logikschaltung erfolgen, die nachträglich nicht mehr verändert werden kann.

Der Datenträger 105 ist beispielsweise durch einen ROM-Speicher, einen EPROM-Speicher oder jeden anderen geeigneten digitalen Speicher gebildet. Der Datenträger 105 wird dem Röntgenstrahler 103 zugeordnet, wie beispielsweise beigelegt oder baulich in das Innere des Röntgenstrahlers 103 integriert.

Der Identifikationscode wird mit einer digitalen Signatur des Herstellers abgesichert, so dass eine Fälschung des Identifikationscodes erkannt wird. Zu diesem Zweck wird überprüft, ob die digitale Signatur für den Identifikationscode gültig ist. Ist diese nicht gültig, kann eine Fehlermeldung ausgegeben werden und der Betrieb des Röntgenstrahlers 103 gesperrt werden.

Eine Abfrageeinrichtung 107 dient zum elektronischen Abfragen des Identifikationscodes aus dem Datenträger 105 des Röntgenstrahlers 103. Das Abfragen des Identifikationscodes kann über eine Steuerleitung oder eine Energieversorgungsleitung 111 des Röntgenstrahlers 103 erfolgen. Die Abfrageeinrichtung 107 ist beispielsweise durch eine elektronische Schaltung gebildet, die mittels digitaler Signale in der Lage ist, den Identifikationscode aus dem Röntgenstrahler 103 auszulesen. Dadurch kann der Identifikationscode aus dem Datenträger 105 ausgelesen werden, so dass dieser von dem Röntgensystem 100 weiterverarbeitet werden kann.

Weiter umfasst das Röntgensystem 100 eine Sperreinrichtung 109 zum Sperren eines Betriebs des Röntgenstrahlers 103, falls der abgefragte Identifikationscode von einem vorgegebenen Identifikationscode abweicht, der durch eine Lizenz erhalten wird. Die Sperreinrichtung 109 ist beispielsweise durch eine elektronische Schaltung gebildet, die in der Lage ist, den Betrieb des Röntgenstrahlers 103 zu verhindern. Das Sperren kann beispielsweise durch ein Unterbrechen einer Energieversorgungsleitung 111 mittels eines Relais stattfinden.

Die Abfrageeinrichtung 107 zum elektronischen Abfragen des Identifikationscodes und die Sperreinrichtung 109 können zudem durch eine Steuersoftware oder ein Computerprogramm implementiert sein. Die Steuersoftware oder das Computerprogramm kann von einem Computer mit einem Speicher und einem Prozessor ausgeführt werden, den das Röntgensystem 100 umfasst. Die Steuersoftware oder das Computerprogramms sind in den Speicher des Computers ladbar.

Die Systemsoftware erkennt und vergleicht somit den Röntgenstrahler 103 anhand des ausgelesenen Identifikationscodes (1.Code) und des vorgegebenen Identifikationscodes der Lizenz (2.Code) und gibt das Röntgensystem 100 im Falle der Übereinstimmung frei. Dadurch kann sichergestellt werden, dass der Röntgenstrahler 103 nur dann betrieben wird, wenn ein gültiger Identifikationscode vorliegt. Zu diesem Zweck wird die herkömmliche Systemsoftware um eine entsprechende Lizenzverwaltung für den Identifikationscode erweitert.

Bei Installation, Inbetriebnahme und Prüfung des Röntgensystems 100 oder des Röntgenstrahlers 103 wird der vorhandene Röntgenstrahler 103 und der elektronisch auslesbare Datenträger 105 angeschlossen und die Konfiguration des Röntgenstrahlers 103 durchgeführt. Nach der Konfiguration, beispielsweise durch Eingabe der Materialnummer und Identifikationscodes des Röntgenstrahlers, werden die Daten verglichen und in das Röntgensystem 100 übernommen.

Eine Lizenz für den Röntgenstrahler 103 kann von einem Lizensierungsserver beantragt werden. Zu diesem Zweck kann der Identifikationscode, die Materialnummer oder die Seriennummer des Röntgenstrahlers 103 angegeben werden. Nach Erhalt der Lizenz wird der vorgegebene Identifikationscode in das Röntgensystem 100 importiert.

Einem Missbrauch der Lizenz unter Angabe der vorher verwendeten Daten kann begegnet werden, indem der Identifikationscode bei jedem Einbau oder Austausch des Röntgenstrahlers 103 überprüft wird. Bei dem Einbau und der darauffolgenden Inbetriebnahme eines neuen Röntgenstrahlers 103 ist eine Einstellung der Betriebsparameter (Tune Up) des Röntgenstrahlers 103 erforderlich, wie beispielsweise eine Einstellung von Strom, Spannung, Zeit und Dosis.

Diese Einstellungsfunktion wird durch eine Bedienoberfläche ausgeführt. Bevor diese Einstellungsfunktion von der Bedienoberfläche freigegeben wird, wird eine Abfrage des Identifikationscodes des Röntgenstrahlers 103 durchgeführt, so dass der Röntgenstrahlers 103 erkannt wird. Die Systemsoftware erkennt den Röntgenstrahler 103 anhand des abgefragten Identifikationscodes (1.Code) und des vorgegebenen Identifikationscodes der Lizenz (2.Code) und gibt das Röntgensystem 100 im Falle der Übereinstimmung frei. Das bedeutet, dass das Röntgensystem 100 (Systemsoftware/Servicesoftware) jeweils vor jeder Neueinstellung der Betriebsparameter selbständig die Identifikationscodes der Lizenz und des Röntgenstrahlers 103 erfragt.

Wird ein Röntgenstrahler 103 mit einem unpassenden Identifikationscode installiert, wird dies erkannt und die Neueinstellung der Betriebsparameter kann gesperrt oder blockiert werden. Ein Hinweis kann in diesem Fall erscheinen, dass ein Servicemitarbeiter des Röntgensystems 100 zu kontaktieren ist.

Zudem kann in diesem Fall eine neue spezielle Lizenz mit einem passenden Identifikationscode von einem Lizenzserver angefordert werden, durch die trotz der anfänglichen Nichterkennung des Röntgenstrahlers 103 das Röntgensystem 100 freigeschaltet wird. Zu diesem Zweck umfasst das Röntgensystem 100 eine Empfangseinheit zum Empfangen eines Identifikationscodes von einem Lizenzserver über ein Datennetz, wie beispielsweise dem Internet. Diese spezielle Lizenz kann zeitlich eingeschränkt gültig sein, z.B. für einen Zeitraum von drei Monaten, in dem der Austausch des Röntgenstrahlers 103 und eine Neueinstellung der Betriebsparameter stattfinden kann. In diesem Zeitraum bleibt das Röntgensystem 100 vorerst freigeschaltet, so dass erst bei einer späteren Neueinstellung der Betriebsparameter eine reguläre Lizenz erforderlich ist.

Nach einem zeitlichen Ablauf einer bestimmten Zeitdauer, wie beispielsweise 12 Monaten, kann eine Erneuerung der speziellen Lizenz gefordert werden. Ein Hinweis auf einen notwendigen Serviceeinsatz wegen einer abgelaufenen Lizenz erscheint. In einer ersten Variante wird festgestellt, dass der Röntgenstrahler 103 ein anderes Fabrikat besitzt als in der Konfiguration angegeben und die Daten des Röntgenstrahlers 103 können automatisch erfasst werden. In einer zweiten Variante erkennt ein Servicemitarbeiter, dass der Röntgenstrahler 103 ein anderes Fabrikat aufweist.

In beiden Fällen kann ebenfalls wieder eine spezielle Lizenz von einem Lizenzserver 113 bereitgestellt werden, um das Röntgensystem 100 mit dem Fremdfabrikat des Röntgenstrahlers 103 freizuschalten. Zudem kann vom Lizenzserver 113 oder Servicecenter eine Abfrage des Identifikationscodes des Röntgenstrahlers 103 durchgeführt werden.

Mit Vergabe der speziellen Lizenz wird in der Systemkonfiguration zusätzlich ein Wert (Flag) gesetzt, der beim Einschalten des Röntgensystems 100 dem Benutzer in der Anzeige einen Hinweis anzeigt. Der Hinweis bezieht sich auf die Verwendung eines anderen Fabrikates des Röntgenstrahlers 103. Der Hinweis kann quittiert werden und verschwindet bis zum nächsten Wiedereinschalten des Systems.

Sollte später der Einbau eines passenden Fabrikates erfolgen, kann wie oben beschrieben bei Inbetriebnahme des Röntgensystems 100 eine Lizenzabfrage mit neuem Identifikationscode, Materialnummer oder Seriennummer gestartet werden.

Dadurch wird sichergestellt, dass nur geeignete Röntgenstrahler 103 in das Röntgensystem 100 eingesetzt werden und die Vorschriften zum Austausch des Röntgenstrahlers 103 eingehalten werden. Beim Austausch des Röntgenstrahlers 103 und der Einstellung der Betriebsparameter (Tune Up) werden besondere Messungen und Protokolle durchgeführt, die in einem technischen Ordner ablegt werden. Durch die ausschließliche Verwendung von passenden Röntgenstrahlern 103 kann die Bildqualität nach einem Austausch des Röntgenstrahlers 103 aufrechterhalten werden.

Ein weiterer Vorteil ist die bekannte Handhabung der Lizenzen und der einfache Nachweis der Verwendung oder Nichtverwendung eines passenden Fabrikats. Daneben ist es ein Vorteil, dass ein anderer nicht-originärer Hersteller des Röntgenstrahlers 103 eine Lizenz erhalten kann. Ein weiterer Vorteil ist, dass eine Laufzeit von 12 oder 24 Monaten der speziellen Lizenz einem Intervall der Wartung entspricht, so dass nach Ablauf der Lizenz eine gleichzeitige Wartung des Röntgensystems 100 von einem Servicemitarbeiter durchgeführt werden kann. Der Schutz mit Hilfe von Datenträger und Lizenz ist für alle Komponenten im Röntgensystem 100 anwendbar.

Fig. 2 zeigt ein Blockdiagram des Verfahrens zum Betreiben des Röntgensystems 100. In einem ersten Schritt S101 wird der Identifikationscode aus dem Datenträger 105 des Röntgenstrahlers 103 elektronisch abgefragt. In einem zweiten Schritt S102 wird der Betrieb des Röntgenstrahlers 103 gesperrt, falls der abgefragte Identifikationscode von dem vorgegebenen Identifikationscode abweicht.

Diese Schritte können jedes Mal bei einer Neueinstellung der Betriebsparameter oder einem Austausch des Röntgenstrahlers 103 durchgeführt werden. Beim Sperren des Betriebes des Röntgenstrahlers 103 kann eine Nachricht über das Internet an den Lizenzserver 113 gesendet werden, die das Röntgensystem 100 spezifiziert. In diesem Fall kann ein Servicemitarbeiter mit der Wartung beauftragt werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Merkmale, die in Bezug auf Verfahrensschritte beschrieben worden sind, können durch entsprechende gegenständliche Merkmale realisiert sein, die zum Ausführen der jeweiligen Verfahrensschritte ausgebildet sind und umgekehrt.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Röntgensystem (100), mit:
- einem Röntgenstrahler (103) mit einem elektronisch auslesbaren Datenträger (105), auf dem ein Identifikationscode gespeichert ist;
- einer Abfrageeinrichtung (107) zum elektronischen Abfragen des Identifikationscodes aus dem Datenträger (105) des Röntgenstrahlers (103), wobei der Datenträger (105) eine elektronische Signatur zum Überprüfen der Integrität des Identifikationscodes umfasst; und
- einer Sperreinrichtung (109) zum Sperren eines Betriebs des Röntgenstrahlers (103), falls der abgefragte Identifikationscode von einem vorgegebenen Identifikationscode abweicht, wobei das Röntgensystem (100) eine Empfangseinheit zum Empfangen des vorgegebenen Identifikationscodes von einem Lizenzserver (113) über ein Datennetz umfasst.

2. Röntgensystem (100) nach Anspruch 1, wobei der Datenträger (105) in dem Röntgenstrahler (103) integriert ist.

3. Röntgensystem (100) nach einem der vorangehenden Ansprüche, wobei der Datenträger (105) ein Speicherchip ist.

4. Röntgensystem (100) nach einem der vorangehenden Ansprüche, wobei der Identifikationscode in dem elektronisch auslesbaren Datenträger (105) festverdrahtet gespeichert ist.

5. Röntgensystem (100) nach einem der vorangehenden Ansprüche, wobei der vorgegebene Identifikationscode eine zeitlich begrenzte Gültigkeit aufweist.

6. Verfahren zum Betreiben eines Röntgensystems (100) mit den Schritten:
- elektronisches Abfragen (S101) eines Identifikationscodes aus einem Datenträger (105) eines Röntgenstrahlers (103), wobei der Datenträger (105) eine elektronische Signatur zum Überprüfen der Integrität des Identifikationscodes umfasst; und
- Sperren (S102) eines Betriebs des Röntgenstrahlers (103), falls der abgefragte Identifikationscode von einem vorgegebenen Identifikationscode abweicht, wobei der vorgegebene Identifikationscode von einem Lizenzserver (113) über ein Datennetz empfangen wird.

7. Verfahren nach Anspruch 6, wobei die Integrität des abgefragten Identifikationscodes anhand einer elektronischen Signatur überprüft wird.

8. Verfahren nach Anspruch 6 oder 7, wobei beim Sperren des Betriebes des Röntgenstrahlers (103) eine Nachricht an den Lizenzserver (113) gesendet wird.

9. Verfahren nach einem der Ansprüche 6 bis 8, wobei das Verfahren vor dem Einstellen der Betriebsparameter des Röntgenstrahlers (103) in dem Röntgensystem (100) durchgeführt wird.

10. Computerprogramm mit Programmcode zum Ausführen des Verfahrens nach einem der Ansprüche 6 bis 9, wenn das Computerprogramm auf einem Computer mit einem Speicher und einem Prozessor ausgeführt wird, der Bestandteil eines Röntgensystems (100) nach einem der Ansprüche 1-6 ist.

## Claims

1. X-ray system (100), comprising:
- an X-ray source (103) with an electronically readable data carrier (105) on which an identification code is stored;
- a query device (107) for electronically querying the identification code from the data carrier (105) of the X-ray source (103), wherein the data carrier (105) includes an electronic signature for checking the integrity of the identification code; and
- a disabling device (109) for disabling operation of the X-ray source (103) in the event of the queried identification code deviating from a predefined identification code, wherein the X-ray system (100) includes a receive unit for receiving the predefined identification code from a license server (113) by way of a data network.

2. X-ray system (100) according to claim 1, wherein the data carrier (105) is integrated in the X-ray source (103).

3. X-ray system (100) according to one of the preceding claims, wherein the data carrier (105) is a memory chip.

4. X-ray system (100) according to one of the preceding claims, wherein the identification code is stored in hardwired fashion in the electronically readable data carrier (105).

5. X-ray system (100) according to one of the preceding claims, wherein the predefined identification code has a temporally limited validity.

6. Method for operating an X-ray system (100), comprising the steps:
- electronically querying (S101) an identification code from a data carrier (105) of an X-ray source (103), wherein the data carrier (105) includes an electronic signature for checking the integrity of the identification code; and
- disabling (S102) operation of the X-ray source (103) in the event of the queried identification code deviating from a predefined identification code, wherein the predefined identification code is received from a license server (113) by way of a data network.

7. Method according to claim 6, wherein the integrity of the queried identification code is checked on the basis of an electronic signature.

8. Method according to claim 6 or 7, wherein a notification is sent to the license server (113) when operation of the X-ray source (103) is disabled.

9. Method according to one of claims 6 to 8, wherein the method is performed prior to setting the operating parameters of the X-ray source (103) in the X-ray system (100).

10. Computer program with program code for carrying out the method according to one of claims 6 to 9 when the computer program is executed on a computer with a memory and a processor which is an element of an X-ray system (100) according to one of claims 1-6.

## Revendications

1. Système (100) à rayons X, comprenant :
- une source (103) de rayons X ayant un support (105) de données, qui est déchiffrable électroniquement et sur lequel est mis en mémoire un code d'identification;
- un dispositif (107) d'appel pour appeler électroniquement le code d'identification du support (105) de données de la source (103) de rayons X, le support (105) de données comprenant une signature électronique pour le contrôle de l'intégrité du code d'identification ; et
- un dispositif (109) de blocage pour bloquer un fonctionnement de la source (103) de rayons X, si le code d'identification appelé s'écarte d'un code d'identification donné à l'avance, le système (100) à rayons X comprenant une unité de réception pour la réception du code d'identification donné à l'avance d'un serveur (113) de licence par l'intermédiaire d'un réseau de données.

2. Système (100) à rayons X suivant la revendication 1, dans lequel le support (105) de données est intégré à la source (103) de rayons X.

3. Système (100) à rayons X suivant l'une des revendications précédentes, dans lequel le support (105) de données est une puce de mémoire.

4. Système (100) à rayons X suivant l'une des revendications précédentes, dans lequel le code d'identification est mis en mémoire à câblage fixe dans le support (105) de données déchiffrable électroniquement.

5. Système (100) à rayons X suivant l'une des revendications précédentes, dans lequel le code d'identification donné à l'avance a une validité limitée dans le temps.

6. Procédé pour faire fonctionner un système (100) à rayons X comprenant les stades :
- on appelle (S101) électroniquement un code d'identification d'un support (105) de données d'une source (103) de rayons X, le support (105) de données comprenant une signature pour le contrôle de l'intégrité du code d'identification; et
- on bloque (S102) un fonctionnement de la source (103) de rayons X, si le code d'identification appelé s'écarte d'un code d'identification donné à l'avance, dans lequel on reçoit le code d'identification donné à l'avance d'un serveur (113) de licence par l'intermédiaire d'un réseau de données.

7. Procédé suivant la revendication 6, dans lequel on contrôle l'intégrité du code d'identification appelé à l'aide d'une signature électronique.

8. Procédé suivant la revendication 6 ou 7, dans lequel, lors du blocage du fonctionnement de la source (103) de rayons X, on envoie un message au serveur (113) de licence.

9. Procédé suivant l'une des revendications 6 à 8, dans lequel on effectue le procédé avant l'établissement des paramètres de fonctionnement de la source (103) de rayons X dans le système (100) à rayons X.

10. Programme d'ordinateur ayant un code de programme pour effectuer le procédé suivant l'une des revendications 6 à 9, lorsque le programme d'ordinateur est réalisé sur un ordinateur ayant une mémoire et un processeur, qui est une partie constitutive d'un système (100) à rayons X suivant l'une des revendications 1 à 6.
